**Europäisches Patentamt**

**European Patent Office**

**Office européen des brevets**

(19)

(11) Veröffentlichungsnummer : **0 411 455 B1**

(12) # EUROPÄISCHE PATENTSCHRIFT

(45) Veröffentlichungstag der Patentschrift :
22.09.93 Patentblatt 93/38

(51) Int. Cl.⁵ : **C07D 201/16**

(21) Anmeldenummer : **90114236.4**

(22) Anmeldetag : **25.07.90**

(54) **Verfahren zur kontinuierlichen Reinigung von Caprolactam.**

(30) Priorität : **02.08.89 DE 3925575**

(43) Veröffentlichungstag der Anmeldung :
**06.02.91 Patentblatt 91/06**

(45) Bekanntmachung des Hinweises auf die
Patenterteilung :
**22.09.93 Patentblatt 93/38**

(84) Benannte Vertragsstaaten :
**BE CH DE ES FR GB IT LI NL**

(56) Entgegenhaltungen :
**EP-A- 0 138 241**
**DD-A- 75 083**
**DE-B- 1 004 616**
**DE-B- 1 253 715**
**DE-B- 1 470 365**

(73) Patentinhaber : **BASF Aktiengesellschaft**
**Carl-Bosch-Strasse 38**
**D-67063 Ludwigshafen (DE)**

(72) Erfinder : **Neubauer, Gerald, Dr.**
**Mozartstrasse 24**
**D-6940 Weinheim (DE)**
Erfinder : **Ritz, Josef, Dr.**
**Osloer Weg 8**
**D-6700 Ludwigshafen (DE)**
Erfinder : **Agar, David, Dr.**
**Eichhornshoehe 37a**
**D-6149 Rimbach (DE)**
Erfinder : **Fischer, Rolf, Dr.**
**Bergstrasse 98**
**D-6900 Heidelberg (DE)**
Erfinder : **Vagt, Uwe, Dr.**
**Im Vogelgesang 89**
**D-6720 Speyer (DE)**
Erfinder : **Fuchs, Hugo, Dr.**
**Egellstrasse 28**
**D-6700 Ludwigshafen (DE)**

**Beschreibung**

An die Reinheit von Caprolactam als Faserrohstoff werden in zunehmendem Maße höhere Anforderungen gestellt. Hierbei ist es erforderlich Kennzahlen des Caprolactams, unter anderem die Permanganatzahl und den Gehalt an flüchtigen Basen, auf einem möglichst niedrigen Wert zu halten.

Aus der DE-PS 1 253 716 ist ein Verfahren bekannt, bei dem man Caprolactam in Gegenwart von Hydrierkatalysatoren in Suspension oder in Rieselfahrweise unter Zusatz von Säuren wie Schwefelsäure hydriert. Nach einem ähnlichen, in der DE-PS 1 253 715 beschriebenen Verfahren setzt man bei der Hydrierung Alkali zu. Dies hat den Nachteil, daß sowohl Säuren als Alkalien wieder aus dem hydrierten Caprolactam abgetrennt werden müssen.

Nach einem anderen, in der DE-PS 1 004 616 beschrieben Verfahren wird zu reinigendes Caprolactam zunächst mit Aktivkohle, dann mit Ionenaustauschern behandelt, anschließend in Gegenwart von Hydrierkatalysatoren in Suspension oder Rieselfahrweise hydriert und dann das hydrierte Caprolactam mit Ionenaustauschern behandelt. Ferner ist aus der DD-PS 75 083 ein Verfahren zur Reinigung von Caprolactam bekannt, wobei man zunächst Caprolactam destilliert und dann Caprolactam gelöst in organischen Lösungsmitteln oder Wasser in Gegenwart eines fest angeordneten Skelett-Katalysators hydriert und anschließend das hydrierte Caprolactam mit Ionenaustauschern behandelt. Die beiden letztgenannten Behandlungsarten sind aufgrund der Vor- und Nachbehandlung außerordentlich aufwendig. Ein Hinweis, wie auf sichere und einfache Weise sowohl Permanganatzahl als auch der Anteil an flüchtigen Basen erniedrigt werden kann, wird nicht gegeben.

Es war deshalb die technische Aufgabe gestellt, ein Reinigungsverfahren für Caprolactam zur Verfügung zu stellen, das wenig aufwendig ist, bei dem der Zusatz von Säuren oder alkalischen Stoffen vermieden wird, eine Vorbehandlung durch Destillation oder mit Ionenaustauschern und desgleichen eine Nachbehandlung mit Ionenaustauschern nicht erforderlich ist und auf sichere Weise die Permanganatzahl und der Gehalt an flüchtigen Basen erniedrigt wird.

Diese Aufgabe wird gelöst in einem Verfahren zur kontinuierlichen Reinigung von Caprolactam durch in Berührung bringen von Caprolactam in wäßriger Lösung mit Wasserstoff bei erhöhter Temperatur und unter erhöhtem Druck in Gegenwart von Hydrierkatalysatoren, wobei man eine 75 bis 95 gew.%ige wäßrige Caprolactamlösung zusammen mit Wasserstoff bei einer Temperatur von 50 bis 95°C unter einem Druck von 1,5 bis 100 bar von unten nach oben über einen in einer rohrförmigen Zone fest angeordneten Palladium- oder Nickelträgerkatalysator leitet und eine Verweilzeit von 10 bis 100 min einhält.

Das neue Verfahren hat den Vorteil, daß zugleich die Permanganatzahl als auch der Anteil an flüchtigen Basen gleichzeitig auf einen niedrigeren Wert gesenkt wird. Weiter hat das neue Verfahren den Vorteil, daß aufwendige Nebenmaßnahmen, wie der Zusatz von Säuren oder Alkalien oder eine zusätzliche Vor- und Nachreinigung mit Aktivkohle oder Ionenaustauschern entfällt. Darüber hinaus hat das neue Verfahren auch den Vorteil, daß die Reinigung mit konzentrierten wäßrigen Lösungen von Caprolactam gelingt, so daß bei der Enddestillation des Caprolactams das Abtrennen von großen Mengen an Wasser entfällt.

Für die Reinigung eignet sich im allgemeinen Caprolactam, das durch Beckmannsche Umlagerung von Cyclohexanonoxim mit Säuren, z.B. konzentrierte Schwefelsäure, Oleum oder Phosphorsäure, oder durch katalytische Umlagerung erhalten wurde. Dabei geht man in der Regel von Cyclohexanonoxim aus, das durch Oximierung von Cyclohexanon mit Hydroxylammoniumsalzen, z.B. Hydroxylammoniumsulfat, unter gleichzeitiger Neutralisation mit Ammoniak, wie in der DE-PS 898 000 beschrieben, durch Oximierung von Cyclohexanon mit Hydroxylammoniumsalzen in Gegenwart von Puffersystemen, wie in der GB-PS 908 859 beschrieben oder durch Oximierung von Cyclohexanon mit Hydroxylammoniumsalzen, z.B. Hydroxylammoniumammoniumsulfat ohne zusätzliche Verwendung von Neutral- oder Puffersalzen bei sinkenden pH-Werten bis zu 0,5 und weniger, wie in der DE-OS 25 08 247 beschrieben, hergestellt worden sind.

Für die Reinigung verwendet man 75 bis 95 gew.%ige wäßrige Lösungen von Caprolactam, insbesondere solche mit einem Gehalt von 80 bis 95 Gew.% Caprolactam.

Besonders vorteilhaft verwendet man wäßrige Lösungen von Caprolactam, die ausgehend von Cyclohexanonoxim, das durch Oximierung von Cyclohexanon mit Hydroxylammoniumammoniumsulfat ohne zusätzliche Verwendung von Neutral- oder Puffersalzen bei sinkenden pH-Werten bis zu 0,5 hergestellt worden sind und das so erhaltene Cyclohexanonoxim durch Beckmannsche Umlagerung in konzentrierter Schwefelsäure und Oleum, anschließende Neutralisation mit Ammoniak, Abtrennen von Rohlactam, Extrahieren des Rohlactams mit einem aromatischen Kohlenwasserstoff wie Benzol oder Toluol und nachfolgendes Abtrennen des organischen Lösungsmittels wie Benzol oder Toluol durch Destillation erhalten worden sind. Hierbei fallen wäßrige Caprolactamlösungen mit einem Gehalt wie oben genannt an.

Die wäßrige Caprolactamlösung wird zusammen mit Wasserstoff von unten nach oben über einen in einer rohrförmigen Zone fest angeordneten Palladium- oder Nickelträgerkatalysator geleitet.

Geeignete Nickelträgerkatalysatoren haben in der Regel einen Gehalt an Nickel von 5 bis 80 Gew.%, bezogen auf Metallgehalt und Träger. Neben Nickel können die Katalysatoren noch aktivierende Zusätze wie Zirkonium, Mangan, Kupfer oder Chrom enthalten, z.B. in Mengen von 1 bis 20 Gew.% bezogen auf die angewandte Menge Nickel. Als Träger verwendet man vorteilhaft Aluminiumoxid, Kieselgel, Tonerden oder Aktivkohle. Besonders geeignet sind Magnesiumsilikat, Aluminiumphosphat, Borphosphat oder Aluminiumoxid.

Geeignete Palladiumträgerkatalysatoren haben vorteilhaft einen Gehalt von 0,01 bis 10 Gew.%, bevorzugt 0,05 bis 5 Gew.% und insbesondere 0,1 bis 2 Gew.% Palladium, bezogen auf die Summe von katalytisch aktivem Metall und Katalysatorträger. Als Träger werden vorteilhaft Aluminiumoxid, Siliciumdioxid, Titandioxid oder Zirkondioxid oder Gemische dieser Oxide verwendet.

Vorteilhaft verwendet man Tränkkatalysatoren, bei denen die katalytisch wirksamen Metalle an der Oberfläche des Trägers angereichert sind. Solche Katalysatoren werden in an sich bekannter Weise durch Behandlung vorgeformter Träger aus den vorgenannten Stoffen in Form von Pellets, Kugeln oder Strängen mit einer wäßrigen Lösung der Metallsalze, z.B. der Nitrate, Trocknen der getränkten Träger, anschließende Calcinierung und Reduktion mit Wasserstoff hergestellt.

Die Palladium- oder Nickelträgerkatalysatoren werden in einer rohrförmigen Zone, z.B. mit einem Verhältnis von Länge:Durchmesser von 10 bis 50:1, fest angeordnet, z.B. als Schüttung, und die wäßrige Caprolactamlösung und Wasserstoff in Sumpffahrweise von unten nach oben über das Bett des fest angeordneten Katalysators geleitet. Nach dem Abkühlen und Entspannen wird aus dem so erhaltenen Reaktionsgemisch, das im wesentlichen aus Caprolactam und Wasser besteht, Caprolactam durch fraktionierte Destillation gewonnen.

Die hydrierende Behandlung führt man bei einer Temperatur von 50 bis 95°C, vorzugsweise 60 bis 95°C, insbesondere 70 bis 90°C, durch. Hierbei hält man einen Druck von 1,5 bis 100 bar, vorteilhaft 5 bis 30 bar, insbesondere 5 bis 20 bar, ein und hält eine flüssige Phase aufrecht.

Wasserstoff wird z.B. in Mengen von 0,0001 bis 1,0 mol je Mol Caprolactam, vorteilhaft 0,001 bis 0,7 mol, insbesondere 0,03 bis 0,3 mol Wasserstoff je Mol Caprolactam verwendet. Hierbei wird eine Verweilzeit von 10 bis 100 min, insbesondere von 15 bis 60 min, eingehalten. Darüber hinaus hat sich eine Belastung von 1,0 bis 6,0, insbesondere 1,5 bis 4 kg Caprolactam je Liter Katalysator und Stunde bewährt.

Nach dem erfindungsgemäßen Verfahren gelingt es, gleichzeitig die Permanganatzahl und den Gehalt an flüchtigen Basen auf einem niedrigen Wert zu halten, ohne daß aufwendige Maßnahmen, wie Behandeln mit Ionenaustauschern und Aktivkohle oder anderen Zusätzen, wie Säuren oder Basen, erforderlich sind.

Das Verfahren nach der Erfindung sei an folgenden Beispielen veranschaulicht. Im folgenden bezeichnen

PTZ  Permanganattitrationszahl
PAZ  Permanganatzahl
FB  flüchtige Basen in Milliäquivalent je Kilogram
UV  UV-Kennzahl
EXT  Extinktion
VWZ  Verweilzeit

Beispiel 1

Das in diesem Beispiel eingesetzte Caprolactam war durch Beckmannsche Umlagerung von Cyclohexanonoxim (hergestellt nach DE 2 508 247) in Oleum, Neutralisation mit Ammoniak, Extraktion mit Benzol und Eindampfen des Extrakts hergestellt worden und wies folgende Kennzahlen auf:

| PTZ | PAZ | UV | EXT | FB |
|---|---|---|---|---|
| 40 | 43.6 | 77.3 | 7.1 | 0.61 |

Ein vertikaler Rohrreaktor (Durchmesser 16 mm, Füllhöhe 35 cm, ölbeheizter Doppelmantel) wurde mit 70 ml (45 g) eines Palladium-Katalysators (Tränkkatalysator, 0,5 Gew.% Pd in feiner Verteilung auf $\gamma$-Aluminiumoxid-Strängen, d=4 mm, l=1 cm) gefüllt. Der Katalysator wurde in 8 Stunden unter schrittweisem Anheben der Temperatur von 80 auf 200°C mit Wasserstoff aktiviert.

Danach wurden unter gleichzeitigem Durchleiten von Wasserstoff (1 bis 10 l/h) bei 50 und 80°C/20 bar Wasserstoff stündlich von unten nach oben (Sumpffahrweise) 50 bis 250 ml einer 80 gew.%igen Caprolactam-Lösung in Wasser durch den Reaktor gepumpt. Vom Kopf des Reaktors gelangte das Hydriergemisch über einen Kühler in einen Abscheider, aus dem das flüssige Reaktionsprodukt und das Abgas ausgeschleust wurden (Versuchsdauer jeweils 2 h). Für das flüssige Reaktionsprodukt wurden PAZ und FB bestimmt. Einzelheiten sind aus Tabelle I zu entnehmen.

Tabelle I

| Nr. | Temp. [°C] | Belastung [kg/1 h] | $H_2$/Lactam [mol/mol] | VWZ [min] | PAZ | FB [meq/kg] |
|---|---|---|---|---|---|---|
| 0 | – | – | – | – | 43.6 | 0.61 |
| 1 | 50 | 1.7 | 0.21 | 28 | 20.0 | 0.46 |
| 2 | 80 | 1.7 | 0.21 | 28 | 10.4 | 0.53 |
| 3 | 80 | 1.7 | 0.04 | 28 | 8.4 | 0.55 |
| 4 | 80 | 1.7 | 0.42 | 28 | 7.6 | 0.44 |
| 5 | 80 | 0.6 | 0.63 | 84 | 5.0 | 0.48 |
| 6 | 80 | 2.9 | 0.13 | 17 | 12.8 | 0.50 |

Vergleichsbeispiel 1

Man verfährt wie in Beispiel 1, Versuch 2, beschrieben, hält jedoch eine Temperatur von 130°C ein, so erhält man einen Austrag mit einer PAZ von 9,8 und FB von 0,79. Der Wert FB ist gegenüber der Ausgangslösung erhöht.

Vergleichsbeispiel 2

Das in diesem Beispiel eingesetzte Caprolactam war durch Beckmannsche Umlagerung von Cyclohexanonoxim (hergestellt nach DE 2 508 247) in Oleum, Neutralisation mit Ammoniak, Extraktion mit Benzol und Eindampfen des Extrakts hergestellt worden und wies folgende Kennzahlen auf:

| PTZ | PAZ | UV | EXT | FB |
|---|---|---|---|---|
| 40 | 43.6 | 77.3 | 7.1 | 0.61 |

Ein vertikaler Rohrreaktor (Durchmesser 16 mm, Füllhöhe 35 cm, ölbeheizter Doppelmantel) wurde mit 70 ml (45 g) eines Palladium-Katalysators (Tränkkatalysator, 0,5 Gew.% Pd in feiner Verteilung auf $\gamma$-Aluminiumoxid-Strängen, d=4 mm, 1=1 cm) gefüllt. Der Katalysator wurde in 8 Stunden unter schrittweisem Anheben der Temperatur von 80 auf 200°C mit Wasserstoff aktiviert.

Danach wurden unter gleichzeitigem Durchleiten von Wasserstoff (1 bis 10 l/h) bei 50, 80 und 130°C/20 bar Wasserstoff stündlich von oben nach unten (Rieselfahrweise) 50 bis 250 ml einer 80 gew.%igen Caprolactam-Lösung in Wasser durch den Reaktor gepumpt. Vom Boden des Reaktors gelangte das Hydriergemisch über einen Kühler in einen Abscheider, aus dem das flüssige Reaktionsprodukt ausgeschleust wurde (Versuchsdauer jeweils 2 h). Für das flüssige Reaktionsprodukt wurden PAZ und FB bestimmt. Tabelle II gibt eine Übersicht zu den erhaltenen Ergebnissen.

Tabelle II

| Nr. | Temp. [°C] | Belastung [kg/1 h] | $H_2$/Lactam [mol/mol] | VWZ [min] | PAZ | FB [meq/kg] |
|---|---|---|---|---|---|---|
| 0 | – | – | – | – | 43.6 | 0.61 |
| 1 | 50 | 1.7 | 0.21 | 28 | 30.8 | 1.47 |
| 2 | 80 | 1.7 | 0.21 | 28 | 13.0 | 1.04 |
| 3 | 130 | 1.7 | 0.21 | 28 | 3.8 | 7.27 |
| 4 | 80 | 1.7 | 0.04 | 28 | 8.8 | 1.31 |
| 5 | 80 | 1.7 | 0.42 | 28 | 10.2 | 1.02 |
| 6 | 80 | 0.6 | 0.63 | 84 | 8.0 | 0.90 |
| 7 | 80 | 2.9 | 0.13 | 17 | 14.4 | 0.68 |

Die Ergebnisse belegen, daß bei Anwendung der Rieselfahrweise deutlich schlechtere Resultate erhalten werden. Es zeigt sich durchweg ein deutlicher Anstieg der Kennzahl für die flüchtigen Basen.

Beispiel 2

Das in diesem Beispiel eingesetzte Caprolactam war durch Beckmannsche Umlagerung von Cyclohexanonoxim (hergestellt nach DE 2 508 247) in Oleum, Neutralisation mit Ammoniak, Extraktion mit Benzol und Eindampfen des Extrakts hergestellt worden und wies folgende Kennzahlen auf:

| PTZ | PAZ | UV | EXT | FB |
|---|---|---|---|---|
| 40 | 43.6 | 77.3 | 7.1 | 0.61 |

Ein vertikaler Rohrreaktor (Durchmesser 16 mm, Füllhöhe 35 cm, ölbeheizter Doppelmantel) wurde mit 70 ml (45 g) eines Palladium-Katalysators (Tränkkatalysator, 0,5 Gew.% Pd in feiner Verteilung auf $\gamma$-Aluminiumoxid-Strängen, d=4 mm, 1=1 cm) gefüllt. Der Katalysator wurde in 8 Stunden unter schrittweisem Anheben der Temperatur von 80 auf 200°C mit Wasserstoff aktiviert.

Danach wurden unter gleichzeitigem Durchleiten von Wasserstoff (5 l/h) bei 80°C/20 bar $H_2$ stündlich von unten nach oben (Sumpffahrweise) 250 ml einer 80 gew.%igen Caprolactam-Lösung in Wasser (= 2,9 g Lactam/ml Kat.h) mit einer Verweilzeit von 17 min durch den Reaktor gepumpt. Vom Kopf des Reaktors gelangte das Reaktionsgemisch über einen Kühler in einen Abscheider, aus dem das flüssige Reaktionsprodukt und das Abgas ausgeschleust wurden (Versuchsdauer 24 h). Für den flüssigen Austrag wurden die PTZ, PAZ, UV-Kennzahl, Extinktion und die flüchtigen Basen bestimmt:

| PTZ | PAZ | UV | EXT | FB |
|---|---|---|---|---|
| 16 | 11.6 | 36.9 | 3.7 | 0.43 |

Das nach Hydrierung erhaltene Extraktlactam wies somit in allen Kennzahlen deutlich verbesserte Werte auf.

Beispiel 3

Das in diesem Beispiel eingesetzte Caprolactam war durch Beckmannsche Umlagerung von Cyclohexanonoxim (hergestellt nach DE 2 508 247) in Oleum, Neutralisation mit Ammoniak, Extraktion mit Benzol und Eindampfen des Extrakts hergestellt worden und wies folgende Kennzahlen auf:

| PTZ | PAZ | UV | EXT | FB |
|---|---|---|---|---|
| 40 | 43.6 | 77.3 | 7.1 | 0.61 |

Ein vertikaler Rohrreaktor (Durchmesser 16 mm, Füllhöhe 35 cm, ölbeheizter Doppelmantel) wurde mit 70 ml (50 g) eines Palladium-Trägerkatalysators (Tränkkatalysator, 0,25 Gew.% Pd in feiner Verteilung auf $\gamma$-Aluminiumoxidkugeln, d=2-4 mm) gefüllt. Der Katalysator wurde in 8 Stunden unter schrittweisem Anheben der Temperatur von 80 auf 200°C mit Wasserstoff aktiviert.

Danach wurden unter gleichzeitigem Durchleiten von Wasserstoff (5 l/h) bei 80°C/20 bar $H_2$ stündlich von unten nach oben (Sumpffahrweise) 125 ml einer 80 %igen Caprolactam-Lösung in Wasser (= 1,4 g Lactam/ml Kat.h) mit einer Verweilzeit von 28 min durch den Reaktor gepumpt. Vom Kopf des Reaktors gelangte das Reaktionsgemisch über einen Kühler in einen Abscheider, aus dem das flüssige Reaktionsprodukt und das Abgas ausgeschleust wurden (Versuchsdauer 24 h). Für den flüssigen Austrag wurden die PTZ, PAZ, UV-Kennzahl, Extinktion und die flüchtigen Basen bestimmt:

| PTZ | PAZ | UV | EXT | FB |
|---|---|---|---|---|
| 21 | 22.6 | 43.5 | 5.1 | 0.51 |

Vergleichsbeispiel 3

Das in diesem Beispiel eingesetzte Caprolactam war durch Beckmannsche Umlagerung von Cyclohexanonoxim (hergestellt nach DE 2 508 247) in Oleum, Neutralisation mit Ammoniak, Extraktion mit Benzol und Eindampfen des Extrakts hergestellt worden und wies folgende Kennzahlen auf:

| PTZ | PAZ | UV | EXT | FB |
|------|------|------|-----|------|
| 40 | 43.6 | 77.3 | 7.1 | 0.61 |

Ein vertikaler Rohrreaktor (Durchmesser 16 mm, Füllhöhe 35 cm, ölbeheizter Doppelmantel) wurde mit 70 ml (45 g) eines Palladium-Katalysators (Tränkkatalysator, 0,5 Gew.% Pd in feiner Verteilung auf $\gamma$-Aluminiumoxid-Strängen, d=4 mm, 1=1 cm) gefüllt. Der Katalysator wurde 2 Stunden bei 80°C mit Wasserstoff aktiviert.

Danach wurden unter gleichzeitigem Durchleiten von Wasserstoff (5 l/h) bei 95°C/20 bar $H_2$ stündlich von oben nach unten (Rieselfahrweise) 125 ml einer 80 %igen Caprolactam-Lösung in Wasser (= 1,4 g Lactam/ml Kat.h) mit einer Verweilzeit von 28 min durch den Reaktor gepumpt. Vom Boden des Reaktors gelangte das Reaktionsgemisch über einen Kühler in einen Abscheider, aus dem das flüssige Produkt und das Abgas ausgeschleust wurden (Versuchsdauer 24 h). Für den flüssigen Austrag wurden die PTZ, PAZ, UV-Kennzahl, Extinktion und die flüchtigen Basen bestimmt:

| PTZ | PAZ | UV | EXT | FB |
|------|------|------|-----|------|
| 18 | 15.6 | 35.7 | 3.5 | 1.26 |

Der Wert für die flüchtigen Basen liegt über dem Ausgangswert.

Beispiel 4

Das in diesem Beispiel eingesetzte Caprolactam war durch Beckmannsche Umlagerung von Cyclohexanonoxim (hergestellt nach DE 2 508 247) in Oleum, Neutralisation mit Ammoniak, Extraktion mit Benzol und Eindampfen des Extrakts hergestellt worden und wies folgende Kennzahlen auf:

| PTZ | PAZ | FB |
|------|------|------|
| 46 | 30.1 | 0.76 |

Ein vertikaler Rohrreaktor (Durchmesser 16 mm, Füllhöhe 35 cm, ölbeheizter Doppelmantel) wurde mit 70 ml (70 g) eines Nickel-Katalysators (56 Gew.% NiO in feiner Verteilung auf Magnesiumsilikat-Pellets, d=5mm) gefüllt. Der Katalysator wurde in 8 Stunden unter schrittweisem Anheben der Temperatur von 80 auf 200°C mit Wasserstoff aktiviert.

Danach wurden unter gleichzeitigem Durchleiten von Wasserstoff (1 bis 10 l/h) bei 50 und 80°C/20 bar Wasserstoff stündlich von unten nach oben (Sumpffahrweise) 50 bis 250 ml einer 80 %igen Caprolactam-Lösung in Wasser durch den Reaktor gepumpt. Vom Kopf des Reaktors gelangte das Hydriergemisch über einen Kühler in einen Abscheider, aus dem das flüssige Reaktionsprodukt und das Abgas ausgeschleust wurden (Versuchsdauer jeweils 2 h). Für das flüssige Reaktionsprodukt wurde jeweils die PAZ bestimmt.

Tabelle III gibt eine Übersicht über die Bedingungen und die erhaltenen Ergebnisse.

Tabelle III

| Nr. | Temp. [°C] | Belastung [kg/1 h] | $H_2$/Lactam [mol/mol] | VWZ [min] | PAZ |
|---|---|---|---|---|---|
| 0 | – | – | – | – | 30.1 |
| 1 | 50 | 1.7 | 0.21 | 28 | 19.0 |
| 2 | 80 | 1.7 | 0.21 | 28 | 12.8 |
| 3 | 80 | 1.7 | 0.04 | 28 | 21.8 |
| 4 | 80 | 1.7 | 0.42 | 28 | 19.0 |
| 5 | 80 | 0.6 | 0.63 | 84 | 11.4 |
| 6 | 80 | 2.9 | 0.13 | 17 | 21.0 |

Vergleichsbeispiel 4

Das in diesem Beispiel eingesetzte Caprolactam war durch Beckmannsche Umlagerung von Cyclohexanonoxim (hergestellt nach DE 2 508 247) in Oleum, Neutralisation mit Ammoniak, Extraktion mit Benzol und Eindampfen des Extrakts hergestellt worden und wies folgende Kennzahlen auf:

| PTZ | PAZ | FB |
|---|---|---|
| 46 | 30.1 | 0.76 |

Ein vertikaler Rohrreaktor (Durchmesser 16 mm, Füllhöhe 35 cm, ölbeheizter Doppelmantel) wurde mit 70 ml (70 g) eines Nickel-Katalysators (56 Gew.% NiO in feiner Verteilung auf Magnesiumsilikat-Pellets, d=5 mm) gefüllt. Der Katalysator wurde in 8 Stunden unter schrittweisem Anheben der Temperatur von 80 auf 200°C mit Wasserstoff aktiviert.

Danach wurden unter gleichzeitigem Durchleiten von Wasserstoff (1 bis 10 l/h) bei 50 bis 130°C/20 bar Wasserstoff stündlich von oben nach unten (Rieselfahrweise) 50 bis 250 ml einer 80 %igen Caprolactam-Lösung in Wasser durch den Reaktor gepumpt. Vom Boden des Reaktors gelangte das Hydriergemisch über einen Kühler in einen Abscheider, aus dem das flüssige Reaktionsprodukt und das Abgas ausgeschleust wurden (Versuchsdauer jeweils 2 h). Für das flüssige Reaktionsprodukt wurde jeweils die PAZ bestimmt.

Tabelle IV gibt eine Übersicht zu den erhaltenen Ergebnissen:

Tabelle IV

| Nr. | Temp. [°C] | Belastung [kg/1 h] | $H_2$/Lactam [mol/mol] | VWZ [min] | PAZ |
|---|---|---|---|---|---|
| 0 | – | – | – | – | 30.1 |
| 1 | 50 | 1.7 | 0.21 | 28 | 23.4 |
| 2 | 80 | 1.7 | 0.21 | 28 | 19.8 |
| 3 | 130 | 1.7 | 0.21 | 28 | 14.0 |
| 4 | 80 | 1.7 | 0.04 | 28 | 29.4 |
| 5 | 80 | 1.7 | 0.42 | 28 | 19.6 |
| 6 | 80 | 0.6 | 0.63 | 84 | 15.2 |
| 7 | 80 | 2.9 | 0.13 | 17 | 31.2 |

Die Ergebnisse belegen, daß bei Anwendung der Rieselfahrweise unter den gleichen Bedingungen wie im Beispiel 4 deutlich schlechtere Resultate erhalten werden. Die Verbesserung in der PAZ erreicht nicht das Niveau, das bei der Sumpffahrweise erreicht wurde.

Vergleichsbeispiel 5 (Suspensionskatalysatoren)

Das in diesem Beispiel eingesetzte Caprolactam war durch Beckmannsche Umlagerung von Cyclohexanonoxim (hergestellt nach DE 2 508 247) in Oleum, Neutralisation mit Ammoniak, Extraktion mit Benzol und Eindampfen des Extrakts hergestellt worden und wies folgende Kennzahlen auf:

| PTZ | UV | EXT | FB |
|-----|-----|-----|------|
| 29 | 55.7 | 4.9 | 0.34 |

200 g einer 95 %igen Caprolactam-Lösung in Wasser und die in der Tabelle angegebene Menge Katalysator wurden in einem 400 ml Glasautoklaven (magnetisch gerührt) vorgelegt, auf die Reaktionstemperatur erwärmt und dann die angegebene Versuchszeit unter 10 bar $H_2$-Druck gerührt. Nach dem Versuchsende wurden jeweils PTZ und FB bestimmt. Einzelheiten sind aus Tabelle V zu entnehmen.

Tabelle V

| Nr. | Katalysator | Temp. [°C] | Zeit [min] | PTZ | FB [meq/kg] |
|-----|-------------|-----------|------------|-----|-------------|
| 0 | – | – | – | 29 | 0.34 |
| 1 | 1600 mg Raney-Nickel | 80 | 30 | 6.3 | 0.38 |
| 2 | 4000 mg 56 % $NiO/MgSiO_3$ | 95 | 60 | 10 | 0.38 |
| 3 | 1600 mg 10 % Pd/Kohle | 80 | 30 | 5.8 | 0.40 |
| 4 | 1600 mg 1.3 % $Ru/Al_2O_3$ | 80 | 60 | 8.5 | 0.60 |

Die Ergebnisse belegen, daß bei Durchführung einer Suspensionshydrierung deutlich schlechtere Resultate erhalten werden. Zwar weisen die Hydrierausträge eine deutliche Verbesserung bei der PTZ auf, jedoch steht dem ein Anstieg der Kennzahl für die flüchtigen Basen gegenüber.

Die Kennzahlen wurden wie folgt bestimmt.

Permanganattitrationszahl (PTZ)

Die Beständigkeit von Caprolactam gegenüber Kaliumpermanganat wird titrimetrisch bestimmt. Die Permanganattitrationszahl (PTZ) ist der Verbrauch an 0.1 N Kaliumpermanganat-Lösung in ml, berechnet auf 1 kg Caprolactam, der bei der Titration schwefelsaurer Lösung gefunden wird.

Permanganatabsorptionszahl (PAZ)

Die Beständigkeit von Caprolactam gegenüber Kaliumpermanganat wird photometrisch bestimmt. Hierzu werden gleiche Mengen einer 0,01 N Kaliumpermanganat-Lösung zu einer 1 % (m/m) wäßrigen Caprolactam-Lösung und zu einer Blindprobe (dest. Wasser) gegeben. Nach 10 Minuten werden die Extinktionen E bei = 420 nm sowohl der Caprolactamprobe als auch der Blindprobe verglichen. Die Permanganatabsorptionszahl berechnet sich aus der gemessenen Extinktion bei 420 nm x 100.

Flüchtige Basen (FB)

(Bestimmung in einer Apparatur nach Parnas; siehe auch ISO-Vorschrift 8661 "Caprolactam for industrial use - Determination of volatile bases content")

Bei einer Destillation im alkalischen Medium werden die flüchtigen Basen aus der Probe freigesetzt (Kjeldahl-Apparatur), in einer 0.02 N Salzsäure aufgefangen und durch Titration mit 0.02 N Natronlauge bestimmt.

$$FB = \frac{(B - a) \times 0.02}{20} \times 1000$$

A = Verbrauch an 0.02 N Natronlauge
B = Verbrauch an 0.02 N Natronlauge für eine Blindbestimmung

UV-Kennzahl (UV)

Die jeweiligen Extinktionen einer 50 % (m/m) wäßrigen Caprolactam-Lösung für 270, 280, 290, 300, 310, 320, 330, 340, 350 und 360 nm werden in einer 10 cm-Küvette bestimmt. Die Summe der Extinktionswerte wird mit 2 multipliziert und ergibt die UV-Kennzahl bezogen auf 100 proz. Caprolactam.

Extinktion (EXT)

Die Extinktion E einer 50 % (m/m) wäßrigen Caprolactam-Lösung wird bei einer Wellenlänge von 290 nm gemessen und auf eine Küvettenlänge 1=1 cm bezogen.

**Patentansprüche**

1. Verfahren zur kontinuierlichen Reinigung von Caprolactam durch in Berührung bringen von Caprolactam in wäßriger Lösung mit Wasserstoff bei erhöhter Temperatur und unter erhöhtem Druck in Gegenwart von Hydrierkatalysatoren, dadurch gekennzeichnet, daß man eine 75 bis 95 gew.%ige wäßrige Caprolactamlösung zusammen mit Wasserstoff bei einer Temperatur von 50 bis 95°C unter einem Druck von 1,5 bis 100 bar von unten nach oben über einen in einer rohrförmigen Zone fest angeordneten Palladium- oder Nickelträgerkatalysator leitet und eine Verweilzeit von 10 bis 100 min einhält.

2. Verfahren nach Anspruch 1, dadurch gekennzeichnet, daß man eine Katalystorbelastung von 1 bis 6 kg Caprolactam je Liter Katalysator und Stunde einhält.

3. Verfahren nach den Ansprüchen 1 und 2, dadurch gekennzeichnet, daß man eine Verweilzeit von 15 bis 60 min einhält.

4. Verfahren nach den Ansprüchen 1 bis 3, dadurch gekennzeichnet, daß man einen Druck von 5 bis 20 bar einhält.

5. Verfahren nach den Ansprüchen 1 bis 4, dadurch gekennzeichnet, daß man eine Temperatur von 70 bis 90°C einhält.

6. Verfahren nach den Ansprüchen 1 bis 5, dadurch gekennzeichnet, daß man einen 0,1 bis 2 gew%.igen Palladium-Trägerkatalysator verwendet.

7. Verfahren nach den Ansprüchen 1 bis 6, dadurch gekennzeichnet, daß man einen 5 bis 80 gew%.igen Nickel-Trägerkatalysator verwendet.

**Claims**

1. A process for the continuous purification of caprolactam by contacting caprolactam in an aqueous solution with hydrogen at elevated temperatures and superatmospheric pressure in the presence of a hydrogenation catalyst by passing a 75-95 % strength by weight aqueous caprolactam solution together with hydrogen at 50-95°C and 1.5-100 bar upward through a fixed-bed supported palladium or nickel catalyst in a tubular zone while maintaining a residence time of from 10 to 100 min.

2. A process as claimed in claim 1, wherein a space velocity is maintained over the catalyst of from 1 to 6 kg of caprolactam per liter of catalyst per hour.

3. A process as claimed in claims 1 and 2, wherein a residence time of from 15 to 60 min is maintained.

4. A process as claimed in claims 1 to 3, wherein a pressure of from 5 to 20 bar is maintained.

5. A process as claimed in claims 1 to 4, wherein a temperature of from 70 to 90°C is maintained.

6. A process as claimed in claims 1 to 5, wherein a 0.1 to 2 % strength by weight supported palladium catalyst is used.

7. A process as claimed in claims 1 to 6, wherein a 5-80 % strength by weight supported nickel catalyst is used.

**Revendications**

1. Procédé de purification en continu de caprolactame par la mise en contact de caprolactame, en solution aqueuse, avec de l'hydrogène à température élevée et sous pression élevée, en présence de catalyseurs d'hydrogénation, caractérisé en ce qu'on envoie de bas en haut, à travers un catalyseur de palladium ou de nickel sur support disposé en lit fixe dans une zone tubulaire, une solution aqueuse de caprolactame à 75-95% en poids, en même temps que de l'hydrogène, à une température de 50 à 95°C et sous une pression de 1,5 à 100 bar, et on maintient une durée de séjour de 10 à 100 mn.

2. Procédé selon la revendication 1, caractérisé en ce qu'on maintient une charge du catalyseur de 1 à 6 kg de caprolactame par litre de catalyseur et par heure.

3. Procédé selon la revendication 1 ou 2, caractérisé en ce qu'on maintient une durée de séjour de 15 à 60 mn.

4. Procédé selon l'une quelconque des revendications 1 à 3, caractérisé en ce qu'on maintient une pression de 5 à 20 bar.

5. Procédé selon l'une quelconque des revendications 1 à 4, caractérisé en ce qu'on maintient une température de 70 à 90°C.

6. Procédé selon l'une quelconque des revendications 1 à 5, caractérisé en ce qu'on utilise un catalyseur à 0,1-2% en poids de palladium sur support.

7. Procédé selon l'une quelconque des revendications 1 à 6, caractérisé en ce qu'on utilise un catalyseur à 5-80% en poids de nickel sur support.